# DEMANDE DE BREVET EUROPEEN

(11) **EP 1 057 417 A1**
(43) Date de publication de la demande: **06.12.2000**
(21) Numéro de dépôt: 00390010.7
(22) Date de dépôt: 25.05.2000
(51) Int. Cl.: A41D 25/00, A61F 5/40

(54) **Cravate suspensoir**

(30) Priorité: 26.05.1999 FR 9906647
(71) Demandeur: Savignol, Jean-Louis, 09230 St. Croix Volvestre (FR)
(72) Inventeur: Savignol, Jean-Louis, 09230 St. Croix Volvestre (FR)

(57) **Abrégé**

La présente invention concerne la cravate suspensoir

Dispositif pour permettre le bien être des parties de l'homme (mise en légère sustentation) et le mouvement permettant la discrétion pour replacer celles-ci.

L'invention concerne un dispositif permettant de soulager sans blessures les parties de l'homme avec un renfort de préférence molletonné le tout solidaire ou non de la cravate (2) considérée.

Elle est constituée d'un orifice (1) réalisé dans un tissus (4) avec un rembourrage molletonnée (3) recouvert du tissus (4) permettant de passer à l'intérieur les parties de l'homme. Lorsque l'utilisateur fait son noeud de cravate, il positionne ses parties en légère sustentation à l'intérieur.

Chaque mouvement de sa main au niveau du noeud lui permet d'un simple geste de replacer ses parties.

Ce dispositif est destiné à placer discrètement avec ou sans mouvement les parties de l'homme.

## Description

La présente invention concerne la cravate suspensoir

Cravate suspensoir qui permet de placer les parties génitales de l'homme à distance. La partie arrière de la cravate est destinée à supporter et dans un molleton les parties du sexe masculin.

Cravate suspensoir caractérisée en ce qu'elle comporte sur la partie arrière de la cravate proprement dite un prolongement d'une longueur plus importante et présente sur ce prolongement un orifice à travers lequel peuvent être passés les parties sexuelles masculines.

La cravate traditionnelle n'a qu'une fonction de décoration sur une chemise par exemple dans sa partie avant. Sa partie arrière étant généralement uniquement utilisée à réaliser le noeud.

La cravate suspensoir permet de remédier à un geste disgracieux de remise des parties génitales dans un slip caleçon ou autre.

La fabrication ou modèle ci-joint comporte une partie traditionnelle avant, et un élément arrière plus large sur le bas et plus étroite sur le haut. Le prolongement arrière dit caché, comporte en sa base (sur le principe identique à la partie avant visible) une dimension identique ou supérieure à la partie avant mais avec un orifice rond ou légèrement ovalisant qui permet de passer les parties génitales de l'homme tout en permettant d'être nouée de manière traditionnelle.

L'intérêt de cette partie arrière est de permettre de soulager le poids des parties par un cordon molletonné disposé à l'intérieur du tissus de la cravate, le deuxième avantage outre le confort permet de replacer discrètement par un mouvement sur le noeud de cravate, les parties se trouvant mal placées dans les plis du pantalon.

Les dessins annexés illustrent l'invention;

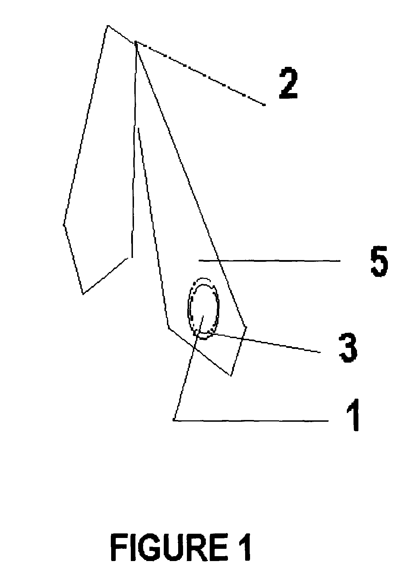

La figure 1 représente la cravate suspensoir dans sa totalité.

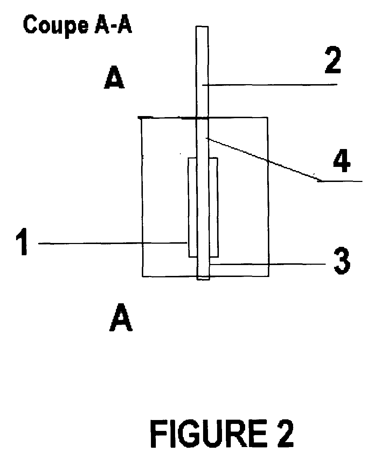

La figure 2 représente la partie arrière de l'invention.

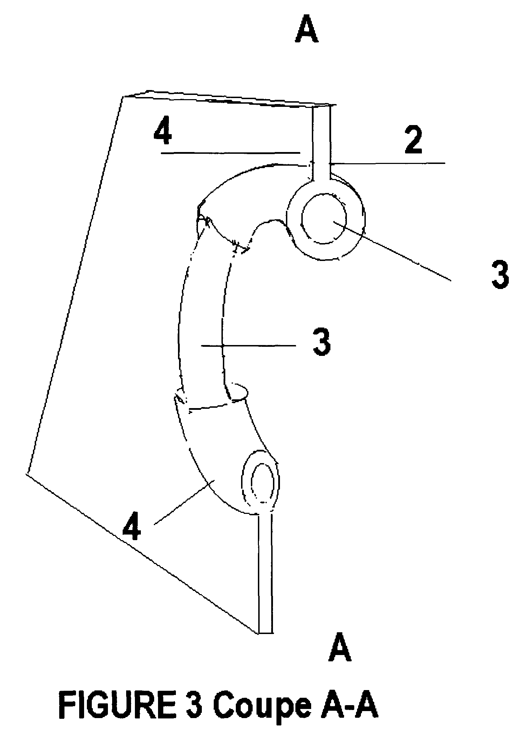

La figure 3 représente en coupe, le dispositif de l'invention.

En référence à ces dessins, le prolongement arrière (5) donc de la cravate est pourvue d'un orifice circulaire (1) taillée à l'origine de la création de la cravate recouverte du même tissu (2) de la cravate ou d'un autre tissus (4) comprenant un molleton (3) afin de ne pas blesser la chair et pour une raison de confort de port.

Selon une variante non illustrée le trou (1) laissant passer les parties peut être légèrement ovalisé.

Selon une deuxième variante le prolongement suspensoir arrière de la cravate (5) peut être réalisée à part pour être rajouté avec un serre cravate traditionnel par exemple.

A titre d'exemple l'orifice (1) de la partie arrière aura un diamètre d'environ 7cm, rappelons que la surface la plus large de la cravate partie avant est d'environ 10cm dans sa pointe basse., dans ce cas la partie arrière de notre cravate aura la même surface.

A titre d'exemple non limitatif, la longueur de la cravate suspensoir sera légèrement supérieure à la cravate traditionnelle d'environ 25cm

## Revendications

1. Cravate suspensoir caractérisé en ce qu'elle comporte sur la partie arrière de la cravate proprement dite un prolongement d'une longueur plus importante et qui présente un orifice (1) à travers lequel peuvent être passés les parties sexuelles masculines, la partie avant de la cravate étant réalisée de manière traditionnelle, l'intérieur de la partie arrière de la cravate suspensoir étant en tissus (2) renforcée par un molleton (3) cousu à l'intérieur du tissu (2 ou 4) de la cravate (5),la longueur totale de la cravate étant supérieure à celle d'une cravate traditionnelle.

2. Cravate suspensoir selon la revendications 1 caractérisé en ce que sa partie arrière est de largeur identique ou supérieure à celle de la partie avant afin de recevoir l'orifice (1) pourvu d'un molleton de renforcement sur toute sa périphérie (3) et recouvert d'un tissus (2ou4).

3. Cravate suspensoir selon la revendication 1 caractérisé en ce qu'elle comporte en outre une partie arrière faisant partie intégrante ou une partie arrière rajoutée et solidarisée par l'intermédiaire d'un serre cravate.

4. Cravate suspensoir selon l'une quelconque des revendication précédentes caractérisée en ce que l'orifice (1) est ovoïde ou circulaire.

5. Cravate suspensoir selon d'une quelconque des revendications précédentes caractérisée en ce que la longueur de la cravate suspensoir est supérieure d'environs 25cm à la longueur d'une cravate traditionnelle.
